# EUROPEAN PATENT APPLICATION

(11) **EP 3 494 876 A1**
(43) Date of publication of application: **12.06.2019**
(21) Application number: 17837294.2
(22) Date of filing: 04.08.2017
(51) Int. Cl.: A61B 5/0285, A61B 5/00

(54) **DEVICE AND METHOD FOR DIAGNOSING BLOOD CIRCULATION DISTURBANCE**

(30) Priority: 05.08.2016 KR 20160100266
(71) Applicant: University Of Ulsan Foundation For Industry Cooperation, Ulsan 44610 (KR)
(72) Inventor: LEEM, Chae Hun, Seoul 04983 (KR)
(74) Representative: v. Bezold & Partner Patentanwälte - PartG mbB
(86) International application number: PCT/KR2017/008464
(87) International publication number: WO 2018/026243

(57) **Abstract**

Disclosed is a device and method for diagnosing a blood circulation disturbance. The device includes a measurement unit configured to measure a first signal and a second signal associated with a cardiac impulse of a patient at two different points in a body of the patient, a matching unit configured to match cycles of the first signal and the second signal, and a diagnosis unit configured to acquire reference points included in the matched cycles of the first signal and the second signal and to diagnose the blood circulation disturbance of the patient using a time difference between the reference points.

## Description

### Technical Field

The present invention relates to a technique for diagnosing a blood circulation disturbance.

### Background Art

Blood vessels are lifelines that supply nutrition to 60 trillion cells in our body. In order to maintain a human life, there is a need for a process in which the blood discharged by the beat of a heart is caused to flow to various parts of a body along an artery without clogging and are returned to the heart via a vein. As a result, oxygen and nutrients can be supplied to the respective tissues of the body, and the wastes consumed through metabolism can be removed. Thus, the health of the blood vessels is directly linked to our health. Serious diseases may be caused when inadvertently managing the blood vessels.

Fat, thrombus, plaque or the like may cause inflammation when they are accumulated on the inner wall of a blood vessel. As inflammatory substances are accumulated, the wall of the blood vessel becomes hard. If the blood vessel becomes narrow due to the accumulation of deposits on the wall of the blood vessel, the supply of blood and oxygen may be impaired and various vascular diseases may appear. Typical examples of vascular diseases include angina pectoris, myocardial infarction, cerebral stroke, and embolism of artery of lower extremity. Body paralysis or sudden death may occur unless a sufficient amount of blood and oxygen is supplied to the heart and the brain which are key organs for life support.

Such vascular diseases progress silently. There is no special subjective symptom until a blood vessel is clogged to some extent or more. Therefore, a fatal result may be caused if the vascular diseases are neglected. For this reason, it is important to diagnose a cardiovascular disease, a cerebrovascular disease and the risk factors of arteriosclerosis at an early stage, even if there is no subjective symptom.

Accordingly, it is urgent to develop a technique for accurately diagnosing a blood circulation disturbance.

### [Prior Art Document]

Korean Patent Laid-Open Publication No. 10-2016-0044271 (April 25, 2016)

### Summary

The present invention provides a technique for accurately diagnosing a blood circulation disturbance.

According to one exemplary embodiment of the present invention, there is provided a device for diagnosing a blood circulation disturbance, comprising: a measurement unit configured to measure a first signal and a second signal associated with a cardiac impulse of a patient at two different points in a body of the patient; a matching unit configured to match cycles of the first signal and the second signal; and a diagnosis unit configured to acquire reference points included in the matched cycles of the first signal and the second signal and to diagnose the blood circulation disturbance of the patient using a time difference between the reference points.

The first signal may be an electrocardiogram measured at a heart of the patient, and the second signal may be a pulse wave measured at a femoral artery, an upper arm, a radial artery, an arm, a wrist, a hand, a leg, an ankle or a foot of the patient.

The first signal may be a pulse wave measured at a carotid artery or a subclavian artery of the patient, and the second signal may be a pulse wave measured at a femoral artery, an upper arm, a radial artery, an arm, a wrist, a hand, a leg, an ankle or a foot of the patient.

The first signal may be a pulse wave measured at a femoral artery of the patient, and the second signal may be a pulse wave measured at a leg, an ankle or a foot of the patient.

The matching unit may be configured to acquire at least one of maximum points of the first signal and the second signal and to match the cycles of the first signal and the second signal based on the maximum point of the second signal generated within one cycle of the first signal from the maximum point of the first signal.

The matching unit may be configured to extract a plurality of candidate minimum points and candidate maximum points from at least one of the first signal and the second signal and to acquire, as the maximum point, one of the candidate maximum points extracted based on an average slope of a section between each of the candidate minimum points and each of the candidate maximum points.

Each of the candidate maximum points may be generated within a predetermined time from a time at which each of the candidate minimum points is generated.

The matching unit may be configured to acquire an average slope of a predetermined section set at a central portion between a time at which each of the candidate minimum points is generated and a time at which each of the candidate maximum points is generated, and to acquire one of the candidate maximum points as the maximum point if the average slope of the predetermined section is larger than an average slope of a section between each of the candidate minimum points and each of the candidate maximum points.

If the first signal is an electrocardiogram measured at a heart of the patient, the reference point of the first signal may be a maximum point of the electrocardiogram.

If at least one of the first signal and the second signal is a pulse wave measured at one point in the body of the patient, the reference point of at least one of the first signal and the second signal may be a minimum point of the pulse wave, and the diagnosis unit may be configured to acquire, as the minimum point of the pulse wave, a point generated closest to a time corresponding to a point at which a slope of a tangent line of the pulse wave is largest among points at which a change value of a slope of the pulse wave is equal to or larger than a predetermined value.

If at least one of the first signal and the second signal is a pulse wave measured at one point in the body of the patient, the reference point of at least one of the first signal and the second signal may be a minimum point of the pulse wave, and the diagnosis unit may be configured to extract a temporary minimum point generated before a maximum point of the pulse wave, divide a section between the extracted temporary minimum point and the maximum point into a predetermined number of equal sections, and acquire, as the minimum point of the pulse wave, a point at which a tangent line having a largest slope in a first section of the equal sections meets with a horizontal line making contact with the temporary minimum point.

The diagnosis unit may be configured to acquire data related to at least one time difference calculated from the reference points of the first signal and the second signal, collect data satisfying a normal distribution having reliability equal to or higher than a predetermined value among the acquired data, and calculate an average value of the collected data to acquire the time difference.

The diagnosis unit may be configured to acquire data related to at least one time difference calculated from the reference points of the first signal and the second signal, collect data in which a Mahalanobis distance is equal to or less than a predetermined value from among the acquired data, and calculate an average value of the collected data to acquire the time difference.

According to another exemplary embodiment of the present invention, there is provided a method for diagnosing a blood circulation disturbance, which is performed by a computing device including at least one processor and a memory for storing at least one program executed by the processor, comprising: measuring a first signal and a second signal associated with a cardiac impulse of a patient at two different points in a body of the patient; matching cycles of the first signal and the second signal; acquiring reference points included in the matched cycles of the first signal and the second signal; and diagnosing the blood circulation disturbance of the patient using a time difference between the reference points.

The first signal may be an electrocardiogram measured at a heart of the patient, and the second signal may be a pulse wave measured at a femoral artery, an upper arm, a radial artery, an arm, a wrist, a hand, a leg, an ankle or a foot of the patient.

The first signal may be a pulse wave measured at a carotid artery or a subclavian artery of the patient, and the second signal may be a pulse wave measured at a femoral artery, an upper arm, a radial artery, an arm, a wrist, a hand, a leg, an ankle or a foot of the patient.

The first signal may be a pulse wave measured at a femoral artery of the patient, and the second signal may be a pulse wave measured at a leg, an ankle or a foot of the patient.

The act of matching cycles may include: acquiring at least one of maximum points of the first signal and the second signal; and matching the cycles of the first signal and the second signal based on the maximum point of the second signal generated within one cycle of the first signal from the maximum point of the first signal.

The act of acquiring at least one of maximum points may include: extracting a plurality of candidate minimum points and candidate maximum points from at least one of the first signal and the second signal; and acquiring, as the maximum point, one of the candidate maximum points extracted based on an average slope of a section between each of the candidate minimum points and each of the candidate maximum points.

Each of the candidate maximum points may be generated within a predetermined time from a time at which each of the candidate minimum points is generated.

The act of acquiring, as the maximum point, one of the candidate maximum points may include: acquiring an average slope of a predetermined section set at a central portion between a time at which each of the candidate minimum points is generated and a time at which each of the candidate maximum points is generated; and acquiring one of the candidate maximum points as the maximum point if the average slope of the predetermined section is larger than an average slope of a section between each of the candidate minimum points and each of the candidate maximum points.

If the first signal is an electrocardiogram measured at a heart of the patient, the reference point of the first signal may be a maximum point of the electrocardiogram.

If at least one of the first signal and the second signal is a pulse wave measured at one point in the body of the patient, the reference point of at least one of the first signal and the second signal may be a minimum point of the pulse wave, and the act of diagnosing the blood circulation disturbance may include acquiring, as the minimum point of the pulse wave, a point generated closest to a time corresponding to a point at which a slope of a tangent line of the pulse wave is largest among points at which a change value of a slope of the pulse wave is equal to or larger than a predetermined value.

If at least one of the first signal and the second signal is a pulse wave measured at one point in the body of the patient, the reference point of at least one of the first signal and the second signal may be a minimum point of the pulse wave, and the act of diagnosing the blood circulation disturbance may include extracting a temporary minimum point generated before a maximum point of the pulse wave, dividing a section between the extracted temporary minimum point and the maximum point into a predetermined number of equal sections, and acquiring, as the minimum point of the pulse wave, a point at which a tangent line having a largest slope in a first section of the equal sections meets with a horizontal line making contact with the temporary minimum point.

The act of diagnosing the blood circulation disturbance may include acquiring data related to at least one time difference calculated from the reference points of the first signal and the second signal, collecting data satisfying a normal distribution having reliability equal to or higher than a predetermined value among the acquired data, and calculating an average value of the collected data to acquire the time difference.

The act of diagnosing the blood circulation disturbance may include acquiring data related to at least one time difference calculated from the reference points of the first signal and the second signal, collecting data in which a Mahalanobis distance is equal to or less than a predetermined value from among the acquired data, and calculating an average value of the collected data to acquire the time difference.

According to a further exemplary embodiment of the present invention, there is provided a computer program stored in a computer-readable recording medium to execute, in combination with hardware: measuring a first signal and a second signal associated with a cardiac impulse of a patient at two different points in a body of the patient; matching cycles of the first signal and the second signal; acquiring reference points included in the matched cycles of the first signal and the second signal; and diagnosing the blood circulation disturbance of the patient using a time difference between the reference points.

According to the embodiments of the present invention, the cycles of the first signal and the second signal can be precisely matched by extracting the maximum points of the first signal and the second signal measured from the body of a patient. Furthermore, when one or more of the first signal and the second signal are pulse waves, the extracted maximum points are checked by comparing the average slopes of the pulse waves. This makes it possible to improve the accuracy of the maximum point extraction even in a pulse wave including noise.

Furthermore, according to the embodiments of the present invention, it is possible to improve the accuracy of a diagnosis result by extracting the reference points from the first signal and the second signal and diagnosing a blood circulation disturbance based on the time difference between the extracted reference points.

In addition, according to the embodiments of the present invention, data corresponding to error occurrence is filtered from a plurality of data related to the time difference between the reference points of the first signal and the second signal, and a blood circulation disturbance is diagnosed using the average value obtained from the filtered data. This makes it possible to acquire a highly reliable diagnosis result.

### Brief Description of the Drawings

FIG. 1 is a block diagram showing a detailed configuration of a blood circulation disturbance diagnosing device according to an embodiment of the present invention.
FIG. 2 is a graph showing an electrocardiogram and waveforms of pulse waves according to an embodiment of the present invention.
FIG. 3 is a flowchart illustrating a method for diagnosing a blood circulation disturbance according to an embodiment of the present invention.
FIG. 4 is a block diagram illustrating and explaining a computing environment including a computing device suitable for use in exemplary embodiments.

### Detailed Description

Hereinafter, specific embodiments of the present invention will be described with reference to the drawings. The following detailed description is provided to enhance the comprehensive understanding of the methods, devices and/or systems described herein. However, this is merely an example and the present invention is not limited thereto.

In the following description, the detailed description of known techniques related to the present invention will be omitted if it is determined that the gist of the present invention may be unnecessarily made obscure by the description. The terms used herein are defined in consideration of the functions in the present invention and may be changed according to the intention of a user, an operator or the like and the custom. Therefore, the definition of the terms should be based on the contents of the subject specification as a whole. The terms used in the detailed description are intended only to describe embodiments of the present invention and should not be limitative. Unless specifically stated otherwise, the expression in a singular form includes the meaning in a plural form. In the following description, the expressions "comprising", "including" and the like are intended to indicate certain features, numbers, steps, operations, elements, or parts or combinations thereof, and should not be construed to preclude the presence or possibility of one or more features, numbers, steps, operations, elements, or parts or combinations thereof other than those described herein.

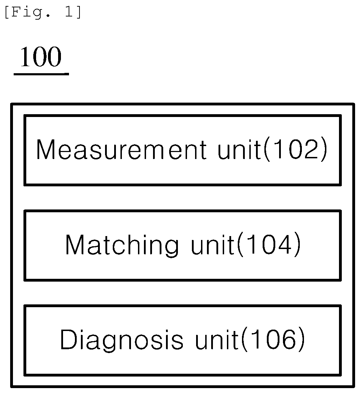

FIG. 1 is a block diagram showing a detailed configuration of a blood circulation disturbance diagnosing device 100 according to an embodiment of the present invention.

In the present embodiments, the pulse wave refers to a wave in which blood propagates in the form of a wave from the heart according to a cardiac impulse. The pulse wave means a pulse transmitted to a peripheral nerve. In this case, the cardiac impulse means the movement of the myocardium that contracts and relaxes to move blood.

The pulse wave may be measured by a pulse wave measuring sensor at the respective parts of the human body. The respective parts of the human body may be, for example, a carotid artery, a subclavian artery, a femoral artery, a brachial artery, a radial artery, an arm, a wrist, a hand, a leg, an ankle, a foot and the like. Meanwhile, in the case of a patient having a symptom of arteriosclerosis, the pulse wave propagation speed increases and, therefore, the shape of the pulse wave changes.

In the present embodiments, an electrocardiogram (ECG) refers to a record of an active current generated in the myocardium according to the cardiac impulse. The electrocardiogram may include Q, R, S and T sections. In addition, the electrocardiogram may be measured by a derivation method derived from the left and right hands, a derivation method derived from the right hand and the left foot, a derivation method derived from the left hand and the left foot, and a derivation method based on unipolar derivation. The above-described derivation methods are well known to those skilled in the art and, therefore, the detailed description thereof will be omitted.

Hereinafter, the configuration of the blood circulation disturbance diagnosing device 100 will be described in detail with reference to FIG. 1. As shown in FIG. 1, the blood circulation disturbance diagnosing device 100 according to an exemplary embodiment of the present invention includes a measurement unit 102, a matching unit 104, and a diagnosis unit 106.

The measurement unit 102 may measure a signal associated with a cardiac impulse from the body of a patient. The term "patient" used herein may be used in a broad sense including not only a patient actually suffering from a blood circulation disturbance but also a subject for which a blood circulation disturbance is to be diagnosed. Signals associated with a cardiac impulse may include one or more of the pulse wave and the electrocardiogram described above. In one example, the pulse wave may be, but is not limited to, a femoral pulse wave measured at the thigh of a patient or a carotid pulse wave measured at the carotid artery of a patient. The pulse wave may be a pulse wave measured at a subclavian artery, an upper arm, a radial artery, an arm, a wrist, a hand, a led, an ankle, a foot and so on. For this purpose, the measurement unit 102 may be provided with a pulse wave measuring sensor.

The measurement unit 102 may measure two signals, i.e., a first signal and a second signal, which are associated with a cardiac impulse, from the body of a patient. Specifically, the first signal may be an electrocardiogram measured at the heart of the patient, and the second signal may be a pulse wave measured at the femoral artery, the upper arm, the radial artery, the arm, the wrist, the hand, the leg, the ankle or the foot of the patient. The first signal may be a pulse wave measured at the carotid artery or the subclavian artery of the patient, and the second signal may be a pulse wave measured at the femoral artery, the upper arm, the radial artery, the arm, the wrist, the hand, the leg, the ankle or the foot of the patient. In addition, the first signal may be a pulse wave measured at the femoral artery of the patient, and the second signal may be a pulse wave measured at the leg, the ankle or the foot of the patient.

The blood circulation disturbance diagnosing device 100 according to an embodiment of the present invention may compare the first signal with the second signal to determine a time period during which a wave is transmitted along a blood vessel. For example, the blood circulation disturbance diagnosing device 100 may measure a transmission time of a wave transmitted from the heart to the thigh according to the cardiac impulse by comparing the electrocardiogram with the pulse wave measured at the thigh. In addition, the blood circulation disturbance diagnosing device 100 may measure a transmission time of a pulse transmitted from the thigh to the ankle according to the cardiac impulse by comparing the pulse wave measured at the thigh with the pulse wave measured at the ankle. Thereafter, the blood circulation disturbance diagnosing device 100 may diagnose a symptom of blood circulation disturbance by comparing the transmission time of a wave transmitted between the heart and the thigh and the transmission time of a wave transmitted between the thigh and the ankle. In the above-mentioned example, a patient having a symptom of arteriosclerosis shows a conspicuous symptom that the blood vessel become harder from the main part toward the distal end part of a body. Therefore, the blood circulation disturbance diagnosing device 100 may acquire a ratio of the transmission time of the wave in each part of the human body and may diagnose a blood circulation disturbance based on each ratio.

The matching unit 104 may match the cycles of the first signal and the second signal. Matching the cycles means specifying mutually corresponding cycles among the cycles of the first signal and the second signal. That is, matching the cycles means a process of interconnecting the cycles generated in the first signal and the second signal by the same cardiac impulse. Specifically, the first signal and the second signal are configured such that the cycle is repeated according to the cardiac impulse. Since the time taken for the wave according to the cardiac impulse to be transmitted to each point of the human body is different, the first signal and the second signal do not record the same waveform. For example, the time at which the maximum point is generated in each of the first signal and the second signal may be different. Accordingly, the matching unit 104 may match a specific cycle of the first signal with a cycle of the second signal generated by the same cardiac impulse.

According to one embodiment, the matching unit 104 may match the cycles of the first signal and the second signal with reference to a maximum point of each of the first signal and the second signal. Specifically, the matching unit 104 may acquire one or more maximum points of the first signal and the second signal and may match the cycles of the first signal and the second signal based on the maximum point of the second signal generated within one cycle of the first signal from the maximum point of the first signal.

On the other hand, the matching unit 104 may acquire a cycle of a cardiac impulse using a fast Fourier transform (FFT). In the present embodiments, the cycle of each of the first signal and the second signal is a signal associated with the cardiac impulse and, therefore, may be the same as the cycle of the cardiac impulse. In one example, the matching unit 104 may convert the data related to the electrocardiogram indicated in terms of a time domain into data in terms of a frequency domain using the FFT. The frequency of the electrocardiogram may be in a range of 40 Hz to 150 Hz. Then, the matching unit 104 may measure the cycle of the cardiac impulse from the frequency of the electrocardiogram thus obtained. However, the cycle of the cardiac impulse varies from moment to moment. Therefore, the time greater than the measured cycle of the cardiac impulse by a predetermined magnitude or more (e.g., 10% of the measured cycle) may be recognized as the cycle of the cardiac impulse.

The matching unit 104 may obtain maximum points of the first signal and the second signal in consideration of the slopes of the first signal and the second signal. For example, the matching unit 104 may extract, as the maximum point, a point at which the measured slope of the electrocardiogram changes from +20 [mV/s] to -20 [mV/s]. However, this is nothing more than an example presented for the sake of understanding and does not limit the method of acquiring the maximum point. If at least one of the first signal and the second signal is a pulse wave, the matching unit 104 may perform a separate algorithm to accurately extract a maximum point of the pulse wave. Since the pulse wave contains a lot of noise due to its characteristics, there is a limitation in accurately extracting the maximum point only by the conventional maximum point acquisition method. Hereinafter, the algorithm for obtaining the maximum point from the measured pulse wave by the matching unit 104 will be described in detail.

First, the matching unit 104 may extract a candidate minimum point and a candidate maximum point from at least one of the first signal and the second signal. The candidate minimum point and the candidate maximum point are a temporary minimum point and a temporary maximum point acquired by a conventional method of extracting a minimum point and a maximum point. In other words, the candidate minimum point and the candidate maximum point may be a set of points respectively estimated as a minimum point and a maximum point. According to one embodiment, the candidate maximum point may be generated within a predetermined time period from the time at which the candidate minimum point has been generated. For example, the candidate maximum point may occur within a time period corresponding to 1/3 of the cycle of the cardiac impulse from the time at which the candidate minimum point has been generated.

Next, the matching unit 104 may acquire a maximum point using an average slope in a section between the candidate minimum point and the candidate maximum point. In particular, the matching unit 104 may acquire an average slope in a section between the candidate minimum point and the candidate maximum point extracted from at least one of the first signal and the second signal. Then, the matching unit 104 may acquire an average slope in a central section set between the time at which the candidate minimum point has been generated and the time at which the candidate maximum point has been generated. In this case, if the average slope of the central section is larger than the average slope in the section between the candidate minimum point and the candidate maximum point, the matching unit 104 may acquire the candidate maximum point as a maximum point.

In one example, the matching unit 104 may divide the time domain between the candidate minimum point and the candidate maximum point into six equal sections. Then, the matching unit 104 may acquire an average slope of at least one of the first signal and the second signal corresponding to the fourth section of the six equal sections. Finally, the matching unit 104 may compare the average slope of the fourth section with the average slope in the section between the candidate minimum point and the candidate maximum point. If the average slope of the fourth section is larger than the average slope in the section between the candidate minimum point and the candidate maximum point, the matching unit 104 may determine that the candidate maximum point is a true maximum point. If the average slope of the fourth section is smaller than the average slope in the section between the candidate minimum point and the candidate maximum point, the matching unit 104 may determine that the candidate maximum point has been erroneously extracted, and may extract a candidate minimum point and a candidate maximum point again.

The diagnosis unit 106 is a module for analyzing the first signal and the second signal to determine whether a patient has a symptom of blood circulation disturbance. Specifically, the diagnosis unit 106 may compare the first signal and the second signal whose cycles have been matched, to acquire a time difference in transmission of a wave generated by a cardiac impulse. The diagnosis unit 106 may determine the presence or absence of a symptom of blood circulation disturbance by measuring a ratio of the time differences in transmission of the waves acquired for the respective parts of a human body.

According to one embodiment, the diagnosis unit 106 may acquire the respective reference points included in the matched cycles of the first signal and the second signal, and may diagnose a blood circulation disturbance of a patient using a time difference between the acquired reference points. For example, the diagnosis unit 106 may determine that there is a symptom of arteriosclerosis, if the ratio of a wave transmission time difference between the main parts of a body to the wave transmission time difference between the distal end parts of a body is equal to or larger than a predetermined value.

According to one embodiment, the reference point of the first signal may be the maximum point of the electrocardiogram if the first signal is an electrocardiogram measured at the heart of a patient. In other words, if the first signal is an electrocardiogram, the diagnosis unit 106 may set the reference point of the first signal to an R section of the electrocardiogram. A detailed description on a process of extracting the R section of the electrocardiogram by the diagnosis unit 106 will be omitted.

However, if the first signal is a pulse wave measured at one point in the body of a patient, the reference point of the first signal may be the minimum point of the pulse wave.

Meanwhile, in the present embodiments, the second signal is a pulse wave and, therefore, the reference point of the second signal may be the minimum point of the pulse wave. Hereinafter, a process of extracting a minimum point of at least one of the first signal and the second signal by the diagnosis unit 106 will be described in detail.

The diagnosis unit 106 may acquire, as the minimum point of at least one of the first signal and the second signal, a point generated closest to the time corresponding to the point at which the slope of a tangent line of at least one of the first signal and the second signal is largest among the points at which a change value of a slope of at least one of the first signal and the second signal is equal to or larger than a predetermined value. In other words, the diagnosis unit 106 may determine that the final point among the points where a second derivative value of at least one of the first signal and the second signal is equal to or larger than a predetermined value in the negative (-) direction of the time from the maximum value of first derivative values obtained by differentiating at least one of the first signal and the second signal with respect to time is the minimum point of at least one of the first signal and the second signal. According to one embodiment, when the slope of at least one of the first signal and the second signal changes from a negative value to a positive value within a predetermined time, the diagnosis unit 106 may determine that the second derivative value is equal to or larger than the predetermined value. Accordingly, when one or more minimum points of the first signal and the second signal are consecutively generated, the diagnosis unit 106 may recognize the last generated minimum point as a true minimum point.

In addition, the diagnosis unit 106 may extract a temporary minimum point generated before the maximum point of at least one of the first signal and the second signal, may divide the section between the extracted temporary minimum point and the maximum point into a predetermined number of (e.g., three) equal sections, may acquire a point at which the slope of a tangent line is largest in the first section of the equal sections, and may specify a point at which the tangent line and the straight line tangent to the temporary minimum point meet, as the minimum point of at least one of the first signal and the second signal. In this regard, the temporary minimum point may mean a minimum point of a graph extracted using a typical algorithm and may be the same as or different from the above-mentioned candidate minimum point.

The diagnosis unit 106 may measure the reference points of the first signal and the second signal in the above-described manner and may acquire data related to the time difference between the reference points. According to one embodiment, the diagnosis unit 106 may acquire a plurality of data related to the time difference. At this time, the diagnosis unit 106 may perform a process of determining whether the acquired plurality of data is data to be collected. That is, the diagnosis unit 106 may filter data related to error occurrence among the acquired plurality of data.

According to one embodiment, the diagnosis unit 106 may selectively collect data that satisfies a normal distribution of 95% confidence. Specifically, the diagnosis unit 106 may acquire an average and a standard deviation from the previously collected data and may update the average and the standard deviation by reflecting the data selectively collected from the average and the standard deviation acquired previously. In one example, if the difference between the newly collected data and the previously acquired average falls within the previously acquired standard deviation, the diagnosis unit 106 may determine that the new data is the data to be collected and may selectively collect the new data.

According to one embodiment, the diagnosis unit 106 may selectively collect data in which Mahalanobis distance is within a predetermined distance (e.g., 2). Specifically, the diagnosis unit 106 may acquire an average and a standard deviation of the collected data. If the value obtained by dividing the difference between the newly acquired data and the previously acquired average by the standard deviation is equal to or less than a predetermined value (e.g., 2), the diagnosis unit 106 may determines that the data is the data to be collected and may selectively collect the data.

Meanwhile, the diagnosis unit 106 may acquire an average value of the data acquired using the normal distribution and an average value of the data acquired using the Mahalanobis distance, respectively, and may compare them to acquire a time difference between the reference points of the first signal and the second signal. Specifically, if the difference between the average value acquired using the normal distribution and the average value acquired using the Mahalanobis distance is less than the predetermined value (e.g., 1/20 of the standard deviation), the diagnosis unit 106 may determine that an average of the two average values is a final time difference.

The accuracy of diagnosis of a blood circulation disturbance may be improved by filtering the data determined to be an error among the acquired data and acquiring an average value of the time difference between the reference points of the first signal and the second signal from the filtered data.

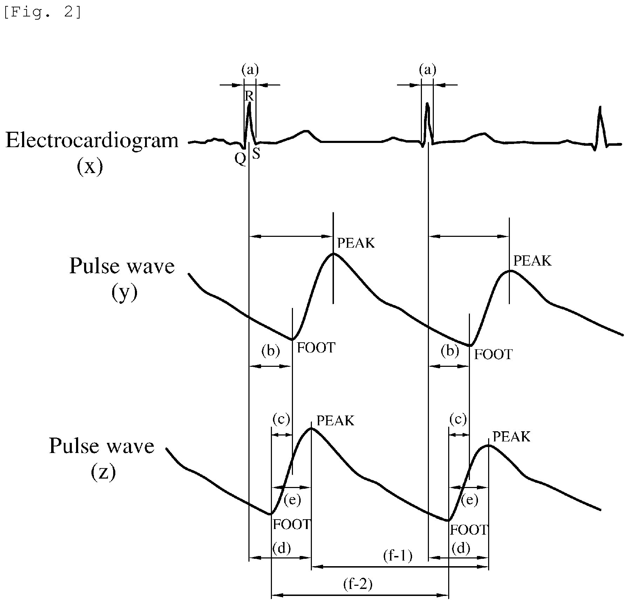

FIG. 2 is a graph showing an electrocardiogram and waveforms of a pulse wave according to an embodiment of the present invention. The blood circulation disturbance diagnosing device 100 according to an embodiment of the present invention may determine the transmission time of a wave transmitted along a blood vessel by comparing the first signal and the second signal. Specifically, the blood circulation disturbance diagnosing device 100 may match the cycles using the maximum points of the first signal and the second signal, and may measure the transmission time of the wave in the blood vessel using the reference points included in the matched cycles of the first signal and the second signal. In this regard, the first signal may be an electrocardiogram measured at the heart of the patient, and the second signal may be a pulse wave measured at the femoral artery, the upper arm, the radial artery, the arm, the wrist, the hand, the leg, the ankle or the foot of the patient. Furthermore, the first signal may be a pulse wave measured at the carotid artery or the subclavian artery of the patient, and the second signal may be a pulse wave measured at the femoral artery, the upper arm, the radial artery, the arm, the wrist, the hand, the leg, the ankle or the foot of the patient. In addition, the first signal may be a pulse wave measured at the femoral artery of the patient, and the second signal may be a pulse wave measured at the leg, the ankle or the foot of the patient.

First, a method of diagnosing a blood circulation disturbance by comparing an electrocardiogram (x) and a pulse wave (y) will be described. At this time, the pulse wave (y) may be a pulse wave measured at the femoral artery, the upper arm, the radial artery, the arm, the wrist, the hand, the leg, the ankle or the foot of a patient. The electrocardiogram (x) is generally composed of P, Q, R, S and T sections. In particular, the R section of the electrocardiogram refers to a section in which the ventricle contracts to discharge blood from the heart. As shown in FIG. 2, the largest electric potential is generated in the R section of the electrocardiogram. That is, the electrocardiogram has a maximum point in the R section (a). On the other hand, in the pulse wave (y), a minimum point (FOOT) and a maximum point (PEAK) are alternately generated as shown in FIG. 2.

The blood circulation disturbance diagnosing device 100 may match the cycles of the respective signals using the maximum point in the R section (a) of the electrocardiogram and the maximum point (PEAK) of the pulse wave (y). According to one embodiment, the blood circulation disturbance diagnosing device 100 may determine that the maximum point of the electrocardiogram (x) and the maximum point (PEAK) of the pulse wave (y) generated within one cycle of a cardiac impulse after generation of the maximum point of the electrocardiogram (x)) correspond to the same cycle. One cycle of the cardiac impulse may be, for example, the difference between the time at which the first maximum point of the electrocardiogram (x) is generated and the time at which the second maximum point of the electrocardiogram (x) is generated. In the above example, the blood circulation disturbance diagnosing device 100 may determine that the first maximum point of the electrocardiogram (x) matches the first maximum point of the pulse wave (y) generated before generation of the second maximum point of the electrocardiogram (x). That is, the blood circulation disturbance diagnosing device 100 may determine that the first maximum point of the electrocardiogram (x) and the first maximum point of the pulse wave (y) are generated by the same cardiac impulse.

The blood circulation disturbance diagnosing device 100 may diagnose a blood circulation disturbance of a patient by using the minimum point (FOOT) of the pulse wave (y). According to one embodiment, the blood circulation disturbance diagnosing device 100 may measure a difference between the time at which the reference point of the electrocardiogram (x) and the time at which the reference point of the pulse wave (y) is generated. At this time, the reference point of the electrocardiogram (x) may be the maximum point of the electrocardiogram (x) and the reference point of the pulse wave (y) may be the minimum point (FOOT) of the pulse wave (y). As described above, the blood circulation disturbance diagnosing device 100 may acquire the time difference (b) between the maximum point in the R section (a) of the electrocardiogram (x) and the minimum point (FOOT) of the pulse wave (y).

Next, a method of diagnosing a blood circulation disturbance by comparing a pulse wave (z) with a pulse wave (y) will be described.

The blood circulation disturbance diagnosing device 100 may match the cycles of the respective signals using the maximum points (PEAKs) of the pulse wave (z) and the pulse wave (y). According to an embodiment, the blood circulation disturbance diagnosing device 100 may determine that the maximum point (PEAK) of the pulse wave (z) and the maximum point (PEAK) of the pulse wave (y) generated within one cycle of a cardiac impulse after the maximum point (PEAK) of the pulse wave (z) is generated correspond to the same cycle. One cycle of the cardiac impulse may be, for example, a difference between the time at which the first maximum point (PEAK) of the pulse wave (y) is generated and the time at which the second maximum point (PEAK) of the pulse wave (y) is generated. In the above example, the blood circulation disturbance diagnosing device 100 may determine that the first maximum point of the pulse wave (z) and the first maximum point (PEAK) of the pulse wave (y) are matched. That is, the blood circulation disturbance diagnosing device 100 may determine that the first maximum point of the pulse wave (z) and the first maximum point of the pulse wave (y) are generated by the same cardiac impulse.

The blood circulation disturbance diagnosing device 100 may diagnose a blood circulation disturbance of a patient using the minimum points (FOOTs) of the pulse wave (z) and the pulse wave (y). According to one embodiment, the blood circulation disturbance diagnosing device 100 may acquire a difference (c) between the time at which the first minimum point (FOOT) of the pulse wave (z) is generated and the time at which the minimum point (FOOT) of the pulse wave (y) is generated.

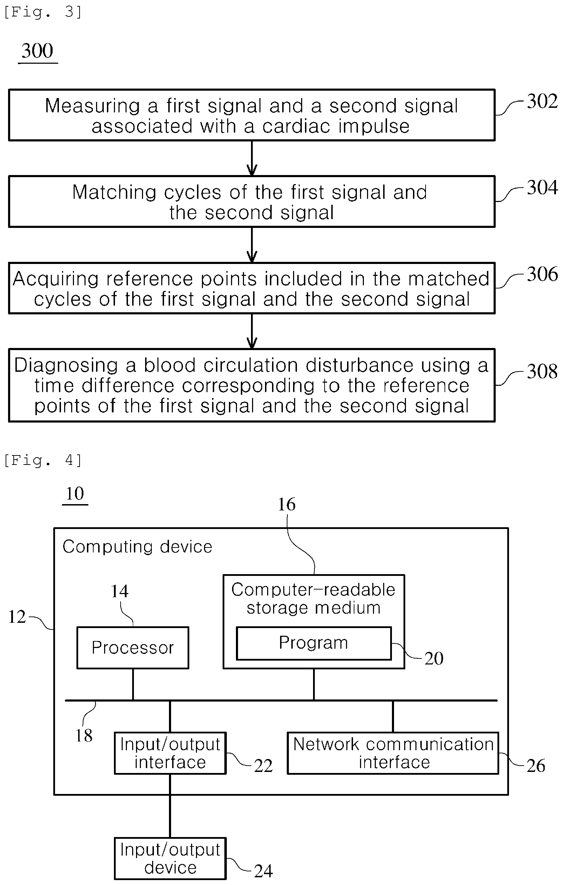

FIG. 3 is a flowchart illustrating a blood circulation disturbance diagnosing method 300 according to an embodiment of the present invention. The method shown in Fig. 3 may be performed, for example, by the blood circulation disturbance diagnosing device 100 described above. In the illustrated flowchart, the method is described as being divided into a plurality of steps. However, at least some of the steps may be performed in different orders, may be combined with other steps and performed together, may be omitted, or may be divided into sub-steps. One or more steps may be added.

The measurement unit 102 may measure a first signal and a second signal associated with a cardiac impulse of a patient at different points in the body of the patient (S302). In one embodiment, the first signal may be an electrocardiogram measured at the heart of the patient, and the second signal may be a pulse wave measured at the femoral artery, the upper arm, the radial artery, the arm, the wrist, the hand, the leg, the ankle or the foot of the patient. Furthermore, the first signal may be a pulse wave measured at the carotid artery or the subclavian artery of the patient, and the second signal may be a pulse wave measured at the femoral artery, the upper arm, the radial artery, the arm, the wrist, the hand, the leg, the ankle or the foot of the patient. In addition, the first signal may be a pulse wave measured at the femoral artery of the patient, and the second signal may be a pulse wave measured at the leg, the ankle or the foot of the patient.

Next, the matching unit 104 may match the cycles of the first signal and the second signal thus measured (S304). Matching the cycles means determining whether each point of the first signal and the second signal is generated by the same cardiac impulse. According to one embodiment, the matching unit 104 may acquire the maximum points of the first signal and the second signal and may match the cycles of the first signal and the second signal based on the maximum point of the second signal generated within one cycle of the first signal from the time at which the maximum point of the first signal is generated. Specifically, the matching unit 104 may extract a candidate minimum point and a candidate maximum point from at least one of the first signal and the second signal, and may acquire a maximum point based on the average slope in the section between the candidate minimum point and the candidate maximum point. Points can be obtained. In this regard, the candidate minimum point and the candidate maximum point are temporary points estimated as a minimum point and a maximum point, and may be acquired through a typical algorithm. In one example, the candidate maximum point may be generated within a predetermined time (e.g., 1/3 of the cycle) from the time at which the candidate minimum point is generated.

The matching unit 104 may execute an algorithm for extracting a true maximum point among one or more candidate maximum points. According to one embodiment, the matching unit 104 may acquire an average slope for a predetermined section set in a central portion between the time at which the candidate minimum point is generated and the time at which the candidate maximum point is generated. If the average slope of the predetermined section is larger than the average slope in the section between the candidate minimum point and the candidate maximum point, the matching unit 104 may acquire the candidate maximum point as the maximum point. For example, the matching unit 104 may divide the section between the candidate minimum point and the candidate maximum point into six equal sections. If the average slope corresponding to the fourth section of the six equal sections is larger than the average slope in the section between the candidate minimum point and the candidate maximum point, the matching unit 104 may determine that the candidate maximum point is a true maximum point.

Next, the diagnosis unit 106 may acquire a reference point within the matched cycles of the first signal and the second signal (S306). At this time, if the first signal is an electrocardiogram measured at the heart of the patient, the reference point of the first signal may be the maximum point of the electrocardiogram. In addition, if at least one of the first signal and the second signal is a pulse wave measured at any one point of the body of the patient, the reference point of at least one of the first signal and the second signal may be the minimum point of the pulse wave.

According to one embodiment, the diagnosis unit 106 may acquire, as the minimum point of at least one of the first signal and the second signal, a point generated closest to the time corresponding to the point at which the slope of a tangent line of at least one of the first signal and the second signal is largest among the points at which a change value of a slope of at least one of the first signal and the second signal is equal to or larger than a predetermined value. In addition, the diagnosis unit 106 may extract a temporary minimum point generated before the maximum point of at least one of the first signal and the second signal, may divide the section between the extracted temporary minimum point and the maximum point into a predetermined number of (e.g., three) equal sections, and may acquire, as the minimum point of at least one of the first signal and the second signal, a point at which a tangent line having a largest slope in the first section of the equal sections meets with a horizontal line making contact with the temporary minimum point.

Next, the diagnosis unit 106 may diagnose a blood circulation disturbance of a patient using the time difference corresponding to the acquired reference point (S308). Specifically, the diagnosis unit 106 may compare the first signal and the second signal whose cycles are matched, to obtain a transmission time difference of the wave by the cardiac impulse. The diagnosis unit 106 may determine the presence or absence of a symptom of a blood circulation disturbance by measuring the ratio of the wave transmission time differences acquired for the respectively parts of the body. According to one embodiment, the diagnosis unit 106 may acquire a plurality of data related to the time difference. In this case, the diagnosis unit 106 may determine whether at least a part of the acquired data is the data acquired due to the occurrence of an error. Specifically, the diagnosis unit 106 may acquires data related to one or more time differences calculated from the reference points of the first signal and the second signal, and may selectively collect the data satisfying a normal distribution having reliability equal to or higher than a predetermined value among the acquired data. In addition, the diagnosis unit 106 may acquire data related to one or more time differences calculated from the reference points of the first signal and the second signal, and may selectively collect the data having a Mahalanobis distance equal to or less than a predetermined value from the acquired data. Then, the diagnosis unit 106 may calculate an average value of the collected data to acquire the time difference.

FIG. 4 is a block diagram for illustrating and explaining a computing environment 10 that includes a computing device suitable for use in the exemplary embodiments. In the illustrated embodiment, each component may have different functions and abilities other than those described below. The computing environment 10 may include additional components in addition to those described below.

The illustrated computing environment 10 includes a computing device 12. In one embodiment, the computing device 12 may be a blood circulation disturbance diagnosing device 100.

The computing device 12 includes at least one processor 14, a computer-readable storage medium 16 and a communication bus 18. The processor 14 may cause the computing device 12 to operate in accordance with the exemplary embodiment discussed above. For example, the processor 14 may execute one or more programs stored in the computer-readable storage medium 16. The one or more programs may include one or more computer-executable instructions. When executed by the processor 14, the computer-executable instructions may cause the computing device 12 to perform operations in accordance with the exemplary embodiment.

The computer-readable storage medium 16 is configured to store computer-executable instructions, program codes, program data and/or other suitable forms of information. The program 20 stored in the computer-readable storage medium 16 includes a set of instructions executable by the processor 14. In one embodiment, the computer-readable storage medium 16 may be a memory (a volatile memory such as random access memory or the like, a non-volatile memory, or any suitable combination thereof), one or more magnetic disk storage devices, optical disk storage devices, flash memory devices, any other form of storage medium that can be accessed by the computing device 12 and can store the desired information, or any suitable combination thereof.

The communication bus 18 interconnects various components of the computing device 12 including the processor 14 and computer-readable storage medium 16.

The computing device 12 may also include at least one input/output interface 22 that provides an interface for at least one input/output device 24, and at least one network communication interface 26. The input/output interface 22 and the network communication interface 26 are connected to the communication bus 18. The input/output device 24 may be connected to other components of the computing device 12 via the input/output interface 22. Examples of the input/output device 24 may include input devices such as a pointing device (such as a mouse or track pad), a keyboard, a touch input device (such as a touch pad or a touch screen), a voice or sound input device, various kinds of sensor devices and/or an imaging device, and output devices such as a display device, a printer, a speaker, and/or a network card. The exemplary input/output device 24 may be included within the computing device 12 as a component of the computing device 12 or may be connected to the computing device 102 as a separate device distinct from the computing device 12.

Meanwhile, the embodiment of the present invention may include a program for performing the methods described herein on a computer, and a computer-readable recording medium including the program. The computer-readable recording medium may include a program command, a local data file, a local data structure, or the like, either alone or in combination. The media may be those specially designed and constructed for the present invention, or may be those commonly used in the field of computer software. Examples of the computer-readable recording medium include hardware devices specially configured to store and execute program commands, such as a magnetic medium such as a hard disk, a floppy disk or a magnetic tape, an optical recording medium such as a CDROM or a DVD, a ROM, a RAM, and a flash memory. Examples of the program may include not only machine language codes such as those produced by a compiler, but also high-level language codes that can be executed by a computer using an interpreter or the like.

While the present invention has been described with reference to the embodiments shown in the drawings, the aforementioned embodiments are nothing more than examples. Those skilled in the art will be able to appreciate that various modifications and other equivalent embodiments may be made from the aforementioned embodiments. Therefore, the true technical protection scope of the present invention should be determined by the technical idea of the appended claims.

## Claims

1. A device for diagnosing a blood circulation disturbance, comprising:
a measurement unit configured to measure a first signal and a second signal associated with a cardiac impulse of a patient at two different points in a body of the patient;
a matching unit configured to match cycles of the first signal and the second signal; and
a diagnosis unit configured to acquire reference points included in the matched cycles of the first signal and the second signal and to diagnose the blood circulation disturbance of the patient using a time difference between the reference points.

2. The device of claim 1, wherein the first signal is an electrocardiogram measured at a heart of the patient, and the second signal is a pulse wave measured at a femoral artery, an upper arm, a radial artery, an arm, a wrist, a hand, a leg, an ankle or a foot of the patient.

3. The device of claim 1, wherein the first signal is a pulse wave measured at a carotid artery or a subclavian artery of the patient, and the second signal is a pulse wave measured at a femoral artery, an upper arm, a radial artery, an arm, a wrist, a hand, a leg, an ankle or a foot of the patient.

4. The device of claim 1, wherein the first signal is a pulse wave measured at a femoral artery of the patient, and the second signal is a pulse wave measured at a leg, an ankle or a foot of the patient.

5. The device of claim 1, wherein the matching unit is configured to acquire at least one of maximum points of the first signal and at least one of maximum points of the second signal and to match the cycles of the first signal and the second signal based on the maximum point of the second signal generated within one cycle of the first signal from the maximum point of the first signal.

6. The device of claim 5, wherein the matching unit is configured to extract a plurality of candidate minimum points and candidate maximum points from at least one of the first signal and the second signal and to acquire, as the maximum point, one of the candidate maximum points extracted based on an average slope of a section between each of the candidate minimum points and each of the candidate maximum points.

7. The device of claim 6, wherein each of the candidate maximum points is generated within a predetermined time from a time at which each of the candidate minimum points is generated.

8. The device of claim 6, wherein the matching unit is configured to acquire an average slope of a predetermined section set at a central portion between a time at which each of the candidate minimum points is generated and a time at which each of the candidate maximum points is generated, and to acquire one of the candidate maximum points as the maximum point if the average slope of the predetermined section is larger than an average slope of a section between each of the candidate minimum points and each of the candidate maximum points.

9. The device of claim 1, wherein if the first signal is an electrocardiogram measured at a heart of the patient, the reference point of the first signal is a maximum point of the electrocardiogram.

10. The device of claim 1, wherein if at least one of the first signal and the second signal is a pulse wave measured at one point in the body of the patient, the reference point of at least one of the first signal and the second signal is a minimum point of the pulse wave, and
the diagnosis unit is configured to acquire, as the minimum point of the pulse wave, a point generated closest to a time corresponding to a point at which a slope of a tangent line of the pulse wave is largest among points at which a change value of a slope of the pulse wave is equal to or larger than a predetermined value.

11. The device of claim 1, wherein if at least one of the first signal and the second signal is a pulse wave measured at one point in the body of the patient, the reference point of at least one of the first signal and the second signal is a minimum point of the pulse wave, and
the diagnosis unit is configured to extract a temporary minimum point generated before a maximum point of the pulse wave, divide a section between the extracted temporary minimum point and the maximum point into a predetermined number of equal sections, and acquire, as the minimum point of the pulse wave, a point at which a tangent line having a largest slope in a first section of the equal sections meets with a horizontal line making contact with the temporary minimum point.

12. The device of claim 1, wherein the diagnosis unit is configured to acquire data related to at least one time difference calculated from the reference points of the first signal and the second signal, collect data satisfying a normal distribution having reliability equal to or higher than a predetermined value among the acquired data, and calculate an average value of the collected data to acquire the time difference.

13. The device of claim 1, wherein the diagnosis unit is configured to acquire data related to at least one time difference calculated from the reference points of the first signal and the second signal, collect data in which a Mahalanobis distance is equal to or less than a predetermined value from among the acquired data, and calculate an average value of the collected data to acquire the time difference.

14. A method for diagnosing a blood circulation disturbance, which is performed by a computing device including at least one processor and a memory for storing at least one program executed by the processor, comprising:
measuring a first signal and a second signal associated with a cardiac impulse of a patient at two different points in a body of the patient;
matching cycles of the first signal and the second signal;
acquiring reference points included in the matched cycles of the first signal and the second signal; and
diagnosing the blood circulation disturbance of the patient using a time difference between the reference points.

15. The method of claim 14, wherein the first signal is an electrocardiogram measured at a heart of the patient, and the second signal is a pulse wave measured at a femoral artery, an upper arm, a radial artery, an arm, a wrist, a hand, a leg, an ankle or a foot of the patient.

16. The method of claim 14, wherein the first signal is a pulse wave measured at a carotid artery or a subclavian artery of the patient, and the second signal is a pulse wave measured at a femoral artery, an upper arm, a radial artery, an arm, a wrist, a hand, a leg, an ankle or a foot of the patient.

17. The method of claim 14, wherein the first signal is a pulse wave measured at a femoral artery of the patient, and the second signal is a pulse wave measured at a leg, an ankle or a foot of the patient.

18. The method of claim 14, wherein the act of matching cycles includes:
acquiring at least one of maximum points of the first signal and at least one of maximum points of the second signal; and
matching the cycles of the first signal and the second signal based on the maximum point of the second signal generated within one cycle of the first signal from the maximum point of the first signal.

19. The method of claim 18, wherein the act of acquiring at least one of maximum points includes:
extracting a plurality of candidate minimum points and candidate maximum points from at least one of the first signal and the second signal; and
acquiring, as the maximum point, one of the candidate maximum points extracted based on an average slope of a section between each of the candidate minimum points and each of the candidate maximum points.

20. The method of claim 19, wherein each of the candidate maximum points is generated within a predetermined time from a time at which each of the candidate minimum points is generated.

21. The method of claim 19, wherein the act of acquiring, as the maximum point, one of the candidate maximum points includes:
acquiring an average slope of a predetermined section set at a central portion between a time at which each of the candidate minimum points is generated and a time at which each of the candidate maximum points is generated; and
acquiring one of the candidate maximum points as the maximum point if the average slope of the predetermined section is larger than an average slope of a section between each of the candidate minimum points and each of the candidate maximum points.

22. The method of claim 14, wherein if the first signal is an electrocardiogram measured at a heart of the patient, the reference point of the first signal is a maximum point of the electrocardiogram.

23. The method of claim 14, wherein if at least one of the first signal and the second signal is a pulse wave measured at one point in the body of the patient, the reference point of at least one of the first signal and the second signal is a minimum point of the pulse wave, and
the act of diagnosing the blood circulation disturbance includes acquiring, as the minimum point of the pulse wave, a point generated closest to a time corresponding to a point at which a slope of a tangent line of the pulse wave is largest among points at which a change value of a slope of the pulse wave is equal to or larger than a predetermined value.

24. The method of claim 14, wherein if at least one of the first signal and the second signal is a pulse wave measured at one point in the body of the patient, the reference point of at least one of the first signal and the second signal is a minimum point of the pulse wave, and
the act of diagnosing the blood circulation disturbance includes extracting a temporary minimum point generated before a maximum point of the pulse wave, dividing a section between the extracted temporary minimum point and the maximum point into a predetermined number of equal sections, and acquiring, as the minimum point of the pulse wave, a point at which a tangent line having a largest slope in a first section of the equal sections meets with a horizontal line making contact with the temporary minimum point.

25. The method of claim 14, wherein the act of diagnosing the blood circulation disturbance includes acquiring data related to at least one time difference calculated from the reference points of the first signal and the second signal, collecting data satisfying a normal distribution having reliability equal to or higher than a predetermined value among the acquired data, and calculating an average value of the collected data to acquire the time difference.

26. The method of claim 14, wherein the act of diagnosing the blood circulation disturbance includes acquiring data related to at least one time difference calculated from the reference points of the first signal and the second signal, collecting data in which a Mahalanobis distance is equal to or less than a predetermined value from among the acquired data, and calculating an average value of the collected data to acquire the time difference.

27. A computer program stored in a computer-readable recording medium to execute, in combination with hardware:
measuring a first signal and a second signal associated with a cardiac impulse of a patient at two different points in a body of the patient;
matching cycles of the first signal and the second signal;
acquiring reference points included in the matched cycles of the first signal and the second signal; and
diagnosing the blood circulation disturbance of the patient using a time difference between the reference points.
